# EUROPEAN PATENT APPLICATION

(11) **EP 1 325 953 A1**
(43) Date of publication of application: **09.07.2003**
(21) Application number: 01965584.4
(22) Date of filing: 12.09.2001
(51) Int. Cl.: C12N 5/06, C12N 5/02

(54) **METHOD OF CULTURING MESENCHYMAL STEM CELLS**

(30) Priority: 12.09.2000 JP 2000000276
(71) Applicant: Kato, Yukio, Hiroshima-shi 732-0062 (JP)
(72) Inventor: KATO, Yukio, Hiroshima-shi, Hiroshima 732-0062 (JP); TSUTSUMI, Shinichi, Hiroshima-shi, Hiroshima 734-0023 (JP); SHIMAZU, Atsushi, Hiroshima-shi, Hiroshima 732-0062 (JP)
(74) Representative: Luderschmidt, Schüler & Partner GbR
(86) International application number: JP0107914
(87) International publication number: WO02022788

(57) **Abstract**

This invention provides a novel method of culturing mesenchymal stem cells whereby a remarkably larger number of mesenchymal stem cells can be obtained compared with the conventional culture methods. This culture method is characterized by adding to a medium a fibroblast growth factor (FGF) as a substance which stimulates the proliferation potency of the mesenchymal stem cells while retaining the pluripotency thereof and prolongs the life. According to the method, mesenchymal stem cells can be cultured at least over 30 generations and, moreover, about 10⁵ to 10⁶ times as much as cell count in the conventional culture methods can be obtained.

## Description

### The technical field

This invention relates to a novel method of culturing mesenchymal stem cells and to the mesenchymal stem cells with the pluripotency obtained by the method. The mesenchymal stem cells of the invention mean the undifferentiated mesenchymal cells with differentiation potential at least into chondrocytes or osteoblasts.

### Background technology

Tissues of a vertebrate, especially mammalian tissues, contain stem cells which have the ability to regenerate cells and tissues in cell regeneration system, for example, to regenerate cells and tissues lost by trauma, disease or aging. Stem cells therefore are found within the tissue or in other tissues that serve as stem cell reservoirs. Bone marrow and periosteum are the major stem cell reservoirs. Bone marrow is the major source of adult hematopoietic stem cells, which are undifferentiated pluripotent cells. It is known that all blood cells (e.g. erythrocyte, leukocyte, lymphocyte, etc.) are differentiated from the hematopoietic stem cells. Many researches have been made on cultivation and differentiation of hematopoietic stem cells, and many substances (growth factors) have been reported which are useful for culturing hematopoietic stem cells and for differentiating and inducing to various blood cells (components).

On the other hand, bone marrow, periosteum and the like contain other stem cells than hematopoietic stem cells, i.e. mesenchymal stem cells, which play an important role for regenerating the mesenchymal tissues such as bone, cartilage, muscle, ligament, tendon, adipose and stroma. Mesenchymal stem cells proliferate as undifferentiated cells and retain the pluripotency, by which can differentiate into such as adipocytes, chondrocytes or osteoblasts. The stem cells can easily be isolated from adult bone marrow and periosteum, and show a stable phenotypic cell form. For example, the mesenchymal stem cells derived from bone marrow retain the character of monolayer *in vitro* cultivation (Pittenger M.F. et al., Science 284, 143-147, 1999).

It was therefore proposed to culture mesenchymal stem cells in large amounts and to differentiate and induce thus obtained undifferentiated cells into cartilage tissue or bone tissue, which can be used for transplantation therapy. Cultivation of bone marrow-derived mesenchymal stem cells was attempted as the first step. The conventional culture methods however cannot produce sufficient amounts of mesenchymal stem cells because the proliferation of said stem cells stops or becomes extremely slow around 15^{th} generation. There is further problem that the differentiation potential to chondrocytes or osteoblasts is lost. In order to induce cartilage or bone tissue from the mesenchymal stem cells and to use them for transplantation therapy, large amounts of said stem cells are required as a start material.

### Disclosure of the invention

This invention aims to provide a method of culturing mesenchymal stem cells which can produce mesenchymal stem cells in remarkably larger amounts compared with conventional culture methods.

This invention also aims to provide mesenchymal stem cells retaining the pluripotency obtained by the method.

As a result of intensive research for solving the above-mentioned problems, it has been found that when mesenchymal stem cells are cultured in a medium containing fibroblast growth factor (FGF), they can be subcultured over 25 generations, based on which finding the invention was completed.

This invention therefore provides a method of culturing mammalian mesenchymal stem cells which is characterized by adding to a medium a substance which stimulates the proliferation potency of mesenchymal stem cells. In this culture method, FGF is useful as the substance which stimulates the proliferation potency of mesenchymal stem cells. The concentration of FGF in the medium is usually from 0.01 to 100 ng/ml, preferably from 0.04 to 10 ng/ml, more preferably from 0.1 to 1 ng/ml.

In the method of this invention, FGF regardless of its origin is applicable so long as it stimulates the proliferation potency of mammalian mesenchymal stem cells. FGF derived from the mammal such as FGF-1 (aFGF) or FGF-2 (bFGF) is desirable. Human bFGF and bovine bFGF are on the market and easily available. FGFs derived from other mammals can also be used in the invention because the receptor is common.

Various FGFs including bFGF have been used like other growth factors for culturing various cells, for example, hematopoietic stem cells (Japanese KOKAI (JP-A) Hei-11(1999)-103856) and chondrocytes (Y. Kato & D. Gospodarowicz, J. Cell Biol., vol 100, 477-485, 1985). It has not been known however that FGF is remarkably effective in retaining the pluripotency of mesenchymal stem cells derived from bone marrow or periosteum, extending their *in vitro* life span and increasing their passage number.

### Mesenchymal stem cells

Mesenchymal stem cells of this invention are defined to be derived from bone marrow and/or periosteum and to be undifferentiated pluripotent cells which can differentiate into mesenchymal tissues such as adipose tissue, cartilage tissue or bone tissue. Mesenchymal stem cells are extractable from any bone marrow or periosteum which contains them. It is desirable to extract them from femur, tibia or pelvis (ilium) because of availability in large amounts and simplicity of extraction. In cases of other mammals than human it is also possible to extract mesenchymal stem cells from ilium, tibia and the like.

In order to extract bone marrow-derived mesenchymal stem cells, any conventional method used for medical treatment and the like can be used. In order to extract stem cells from bone marrow of other mammals than human by a laboratory-method, both ends of bone (e.g. femur, tibia) are cut, the inside of bone is washed by a medium suitable for culturing mesenchymal stem cells, and mesenchymal stem cells can be obtained from the emerged medium. A practical procedure is as follows:
(1) The both ends of rabbit femur or tibia are cut. Subsequently the inside of bone marrow is washed by a medium (e.g. DMEM medium) to which antibiotics (penicillin, streptomycin, etc.) and heparin can optionally be added.
   In the case of human ilium, bone marrow liquid is extracted from the ilium bone marrow.
(2) The medium used for washing is centrifuged for about 3 minutes at 300 × g to precipitate bone marrow cells. The obtained cells which are diluted with a suitable medium (e.g. DMEM medium containing 10% FBS) to a suitable concentration are seeded onto a cell culture plate. They are cultured for three days to produce bone marrow-derived mesenchymal stem cells that adhere on the culture plate.

In order to extract the stem cells from the periosteum, a well-known method can be used [M. Iwasaki et al., Endocrinology 132, 1603-1608 (1993); J. Fang & B. K. Hall, Developmental Biol. 180, 701-712 (1996); S. Bahrami et al., The Anatomical Record 259, 124-130 (2000)]. An example of method is given in the following.

(1) Periosteum is exposed by exfoliating soft tissues such as connective tissue and the like from rabbit femur or tibia, cut in a suitable size and extracted. The extracted periosteum is chopped finely and treated with collagenase at 37°C.

(2) Cells are subsequently isolated by pipetting or other suitable method and collected by filtration or centrifugal separation. Thus obtained cells are counted, diluted with a suitable medium (e.g. DMEM medium containing 10% FBS) to a suitable concentration and seeded onto a cell culture plate. They are cultured for three days to produce periosteum-derived mesenchymal stem cells that adhere on the culture plate.

By such method, mesenchymal stem cells of about 10⁴-10⁵ cell counts can be obtained. Although thus obtained mesenchymal stem cells can directly be seeded and cultured, it is common to use them after culturing for 10-20 days in a suitable medium. The cells can be cryo-preserved.

### Method of culturing mesenchymal stem cells

The stem cells obtained as mentioned above are cultured in a medium which is suitable to culture the cells and contains bFGF in an amount of 0.01 to 100 ng/ml, preferably 0.04 to 10 ng/ml, more preferably 0.1 to 1 ng/ml, for example, 1 ng/ml. Cultivation can be carried out under conditions suitable for culturing mammal cells, usually in the presence of 5% CO₂ at 37°C. An example of cultivation is given in the following.

(3) The mesenchymal stem cells adhering on the cultivation plate as mentioned above are cultured in a suitable medium (e.g. DMEM medium containing 10% FBS) in the presence of 5% CO₂ at 37°C.

(4) The medium is renewed every 3 days. bFGF will be added from day 5 to the medium in an amount of 1 ng/ml.

The subculture of the stem cells can be performed by a suitable method known in the field of cell culture. For example, cells are collected from the plate of the primary culture which are close to confluence, seeded in a suitable medium containing bFGF and cultured under the similar conditions as primary culture. When cells approach confluence, they are subcultured. An example of subculture is given in the following.

(5) The primary culture mentioned in above (4) becomes close to confluence in around 10 days. This plate is treated with trypsin (e.g. 0.05%) + EDTA (e.g. 0.2mM). Cells are collected from the plate and counted.

(6) The cultured stem cells are seeded at a density of 5 × 10³ cells/cm² in a medium containing bFGF (1 ng/ml), cultured and subcultured before cells become confluent.

(7) Subculture is performed by repeating the procedure of above (6).

According to the culture method of the invention the proliferation of mesenchymal stem cells continues to 15 generations, preferably 25 generations, more preferably over 30 generations, or for more than 16 days, preferably more than 20 days, more preferably more than 30 days, further preferably more than 40 days and most preferably more than 50 days to produce extremely high numbers of stem cells. Thus cultured stem cells can be differentiated into various mesenchymal tissues such as cartilage tissue or bone tissue. In order to obtain chondrocytes (tissue) from the cultured mesenchymal stem cells, the stem cells are treated with trypsin, isolated by centrifugal separation etc., transferred to a suspension culture system, an agarose culture system or an alginic acid culture system, and cultured to induce chondrocytes in a medium suitable for chondrogenic differentiation, for example, a medium described by M. F. Pittenger et al., Science 284, 143-147, 1999.

In order to obtain osteocytes (tissue) from the mesenchymal stem cells, the stem cells are treated with trypsin, isolated by centrifugal separation etc. and cultured to induce osteocytes in a medium suitable for osteogenic differentiation, for example, a medium described by M. F. Pittenger mentioned above.

The invention is explained more in detail by examples by which the invention is not restricted.

### Best mode for working the invention

### Examples

### Example 1 Cultivation of mesenchymal stem cells

### 1) Collection of mesenchymal stem cells

### 1-1) Collection of stem cells from bone marrow

Femurs and tibias of four-week-old rabbits were freed from muscles, ligament and the like, and extracted. The both ends were cut off. The inside of bone marrow was washed with DMEM medium (containing 32 unit/ml penicillin, 50 µg/ml streptomycin and 6000 units/ml heparin). The emerged medium was subjected to centrifugal separation (at 300 × g, for 3 minutes) to precipitate stem cells. They were diluted with DMEM medium containing 10% FBS, seeded at 2 x 10⁸ cells (containing erythrocytes)/10 cm culture plate and cultured for three days in the presence of 5% CO₂ at 37°C to produce adhering cells (about 2000 cells/culture plate), which were used as the bone marrow-derived mesenchymal stem cells.

### 1-2) Collection of stem cells from periosteum

Femurs and tibias of four-week-old rabbits were feed from soft tissues such as connective tissue to expose periosteum. The periosteum was excised and extracted in a size of about 5 x 5 mm. After removing soft tissues, the periosteum was chopped finely and treated with 0.1% collagenase for 120 minutes at 37°C. Subsequently, cells were liberated by pipetting, collected by filtering and then washed 3 times with PBS. The cells were diluted with DMEM medium containing 10% FBS, counted and seeded at 2 × 10⁵ cells/10 cm culture plate. The cells were cultured for 3 days to produce adhering cells which were used as periosteum-derived mesenchymal stem cells.

### 2) Cultivation of mesenchymal stem cells

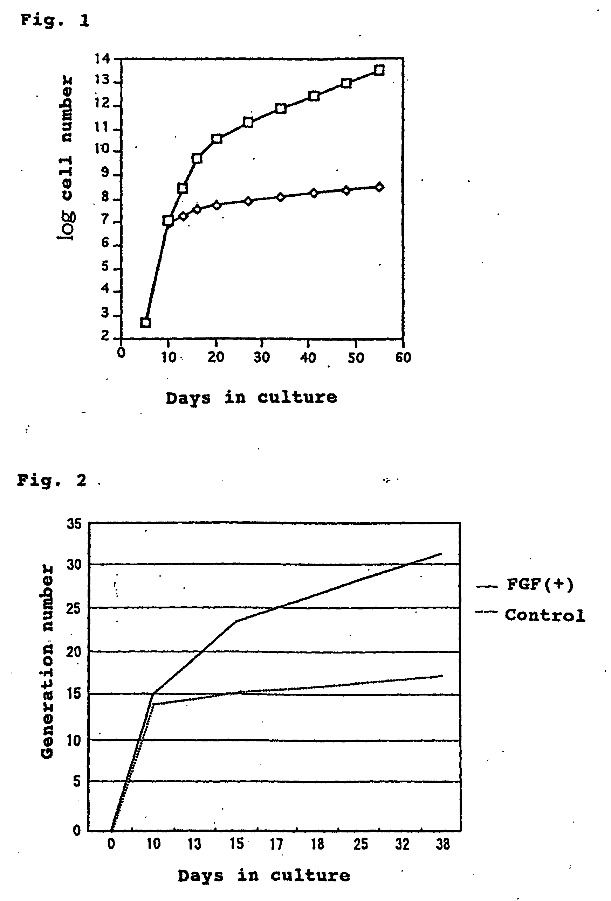

The mesenchymal stem cells extracted from bone marrow were cultured in DMEM medium containing 10% FBS, during which the medium was renewed every 3 days. bFGF was added in case of periosteum-derived cells from 2^{nd} day, in case of bone marrow-derived cells from 5^{th} day to the medium in an amount of 1 ng/ml, and no bFGF was added to control group. The both groups apgroached confluence around 10^{th} day. The plates were treated with 0.05% trypsin + 0.2mM EDTA for 5 minutes to isolate the grown stem cells. After counting the number of cells by Coulter counter (Z1 single, produced by Coulter Company), the cells were seeded at a density of 5000 cells/cm². bFGF was also added in the subculture in an amount of 1 ng/ml. The cultivation results of mesenchymal stem cells of bFGF-added group and control group are shown in graphs of Fig. 1 (cell number/days in culture) and Fig. 2 (generation number/days in culture).

With the conventional culturing method (control group) in which no bFGF was added, the cell growth was active up to 10^{th} day from the start of culturing (around 15 generations) but then stopped. On the other hand with the culturing method of this invention, the cell growth continued even after 30^{th} day (about 30 generations) although the cell growth speed lowered in some degree around 20^{th} day. The mesenchymal stem cells produced by the culturing method of this invention in 50 days was 10⁵ times the cell count of the control group (see Fig. 1 and Fig. 2).

### Example 2 Chondrogenic differentiation

The mesenchymal stem cells cultured in Example 1 (bFGF added-group and control group, 16^{th} day from the start of culturing) were treated with 0.05% trypsin + 0.2mM EDTA for 5 minutes to isolate mesenchymal stem cells. Then 2.5 × 10⁵ cells were transferred to 15ml test tube (made of polypropylene) and subjected to centrifugal separation at 1000 × g for 5 minutes to remove DMEM medium containing FBS. The precipitated cells were mixed with a chondrogenic differentiation medium having the following composition and cultured in centrifugation tubes.

| Chondrogenic differentiation medium | |
|---|---|
| High glucose α-MEM medium | |
| 10 ng/ml TGFβ1 | |
| 100 nM Dexamethasone | |
| 50 µg/ml Ascorbic acid-2-phosphate | |
| 100 µg /ml Sodium pyruvate | |
| ITS-plus | 6.25 µg /ml Transferrin |
| | 6.25 µg/ml Insulin |
| | 6.25 ng/ml Selenic acid |
| | 5.33 µg/ml Linoleic acid |
| | 1.25 mg/ml Bovine serum albumin |

The mesenchymal stem cells were cultured in the above-mentioned medium in the presence of 5% CO² at 37°C. After 24 hours from the start of culturing, the cells formed spherical pellets. Renewing the medium every 2 days the cultivation was continued for 14 days.

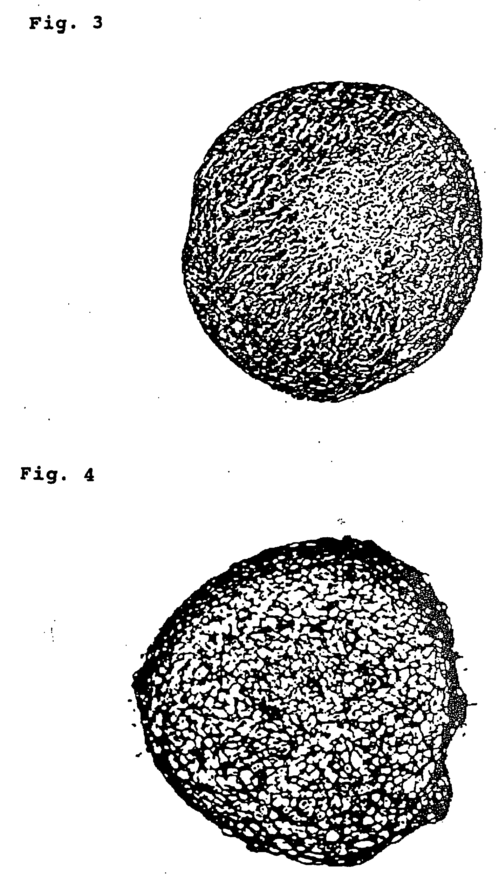

The slides of the pellet cultures before cultivation and after cultivation were prepared and stained with Toluidine Blue. Judging from the shape of cell mass and the stainability of proteoglycan, an extracellular matrix, it was confirmed with the bFGF-added group that the undifferentiated state was observed before the induction (Fig. 3) but the cartilage tissue induction was observed after the cultivation (Fig. 4). On the other hand chondrocytes were not observed with the control group. Cell shape of the mesenchymal stem cells before chondrogenic differentiation was retained with bFGF-added group, but fibroblast-like cells were observed with the control group. These results are summarized in the following table.

**Table 1**

| | Retention of shape of stem cells before induction | Chondrogenic differentiation | |
|---|---|---|---|
| | | Chondrogenic differentiation | Ratio (%) |
| bFGF-added | ++ | +++ | 100 |
| group | | | |
| | | | |
| Control | - | - | 0 |
| group | | | |

### Example 3 Osteogenic differentiation

The mesenchymal stem cells (bFGF added-group, control group) were collected on 16^{th} day after cultivation as in Example 2 and transferred into an osteogenic differntiation medium having the following composition.

### Osteogenic differentiation medium

αMEM medium
10% FBS
100 nM Dexamethasone
10 mM β-Glycerophosphoric acid

### 50µg/ml Ascorbic acid 2-phosphate

The stem cells were cultured in the above-mentioned medium in the presence of 5% CO₂ for 12 days at 37°C, renewing the medium every 2 days.

When the cells after cultivation were stained by Alizarin Red, the calcification was observed with bFGF added-group.

On the other hand with the control group the calcification was remarkably low compared with the bFGF-added group. The results are summarized in the following table.

**Table 2**

| | Osteogenic differentiation |
|---|---|
| bFGF-added | +++ |
| group | |
| | |
| Control group | + |

### Example 4 Effects on proliferation of various mesenchymal stem cells

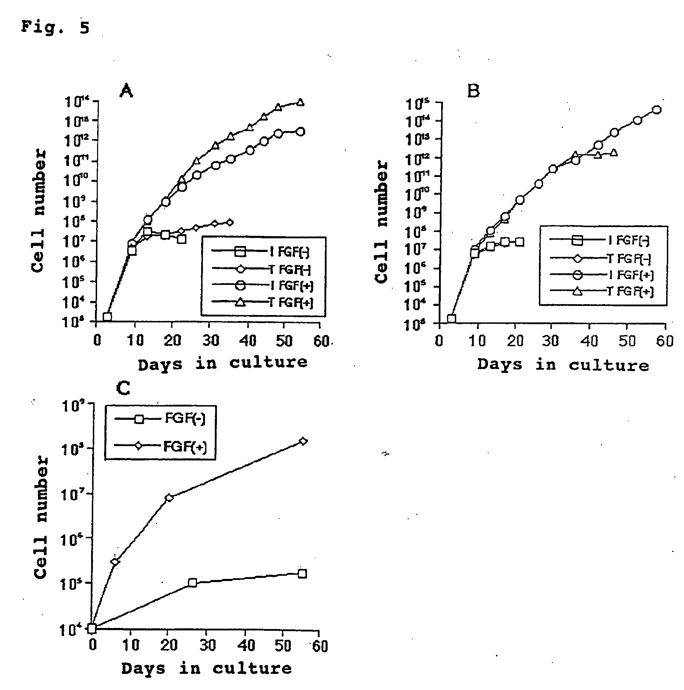

The bone marrow-derived mesenchymal stem cells from rabbit ilium and tibia were obtained as in Example 1. The bone marrow-derived mesenchymal stem cells from human tibia were obtained from BioWhittaker Inc. (Walkersville, MD). The rabbit-derived mesenchymal stem cells were seeded in at 2 × 10⁸ cells (containing erythrocyte)/10cm cultivation plate. The human-derived mesenchymal stem cells were seeded at 10³ cells/cm². They were cultured in the presence of bFGF (1 ng/ml) or absence. Subculture was performed by seeding the rabbit-derived mesenchymal stem cells at 5 × 10³ cells/cm² and the human-derived mesenchymal stem cells at 10³/cm² (see Example 1). Cultivation was carried out twice for each mesenchymal stem cells independently from each other. The results are shown in Fig. 5.

As shown in Fig. 5, the addition of bFGF increased the growth rate and the life span of both mesenchymal stem cells derived from rabbit ilium (I) and tibia (T) (see A and B). The mesenchymal stem cells derived from human tibia showed an increase of growth rate and an extension of life in human serum-containing medium (see C).

The same results were obtained also with mesenchymal stem cells derived from dog ilium (results are not shown).

### Example 5 Verification of the chondrogenic differentiation potential of cultured mesenchymal stem cells

### 1) Histochemical analysis

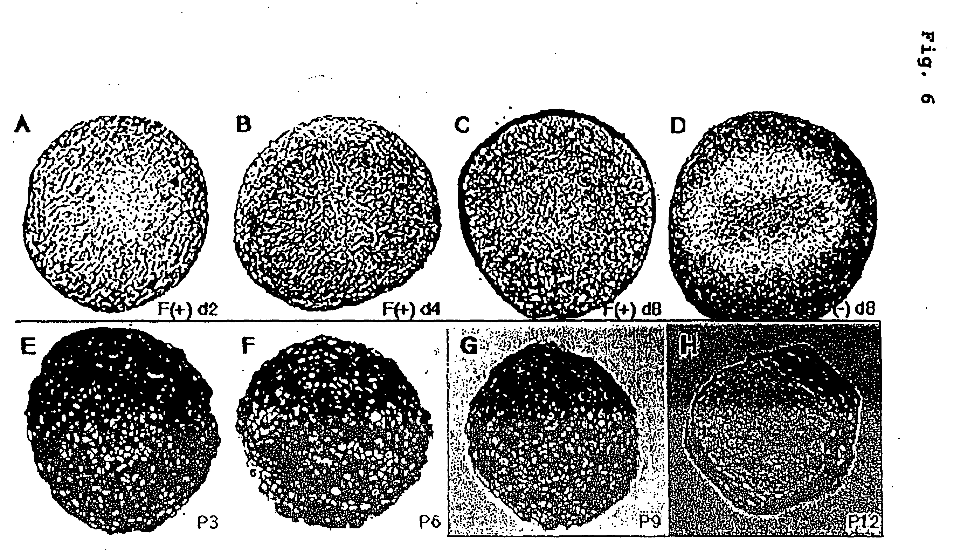

The mesenchymal stem cells extracted from a four-week-old rabbit were grown in the presence of bFGF (FGF(+)) or absence (FGF(-)), subcultured 3 times, transferred to the chondrogenic differentiation medium used in Example 2, cultured for 2, 4 or 8 days and stained with Toluidine Blue. The differentiation into chondrocytes was not observed in 2 days from the bFGF chondrogenic differentiation (Fig. 6 - A) but spherical cells (chondrocytes) were observed on the surface of pellets in 4 days (Fig. 6 - B). In eight days all mesenchymal stem cells of FGF(+) became spherical (Fig. 6 - C). On the other hand, mesenchymal stem cells of FGF(-), chondrogenic differentiation was notably late as compared with the cells of FGF(+) (Fig. 6 - D).

The mesenchymal stem cells of FGF(+) which were passaged 3, 6, 9 or 12 times were transferred to the chondrogenic differentiation medium, cultured for 16 days and stained with Toluidine Blue. Although a cartilage-like tissue was observed in almost all cells of 3^{rd} - 9^{th} passage cultures, the amount of cartilage proteoglycan, which was stained with Toluidine Blue, decreased with the increase in the passage number (Fig. 6 - E ∼ G). Only a part of cells of 12^{th} passage turned into chondrocytes (Fig. 6 - H). The results indicate that although FGF promotes chondrogenic differentiation of mesenchymal stem cells but the differentiation potential decreases with the increase of passage.

### 2) Biochemical analysis and analysis with marker genes

The mesenchymal stem cells subcultured to 3, 6, 9 or 12^{th} generation in FGF(+) or FGF(-) were tested for glucosamine content and alkaline phosphatase activity and analyzed for the amount of mRNAs of type II collagen and type X collagen, which are molecular markers of cartilage.

### (1) Method

The glycosaminoglycan content was measured by the method of Bitter and Muir (Anal. Biochem. 4 (1962), 330-334). Alkaline phosphatase activity in cultured cells was measured by the method of Bessey (J. Biol. Chem. 164 (1946), 321-329). RT-PCR was performed to detect mRNA as follows.

### RT-PCR

Total RNA was extracted from cultured cells using ISOGEN (Nippon Gene, Tokyo, Japan). The first-strand cDNA was synthesized using SUPERSCRIPT II RNase H-reverse transcriptase (Life Technologies, Inc. Rockville, MD) from 1µg of total RNA. Using synthesized cDNA as a template, PCR amplification was carried out under the following PCR conditions: denaturation at 94°C for 30 seconds; the primer extension at 65°C for 1.5 minutes in 25 or 30 cycles. Thus obtained PCR products were separated by 1% agarose gel electrophoresis and detected by ethidium bromide staining. The following set of primers was used.

### Primers

Rabbit type II collagen 5'-CATACCGGTAAGTGGGGCAAGACTG-3' (SEQ ID NO: 1) and

5'-TGCCCAGTTCAGGTCTCTTA-3' (SEQ ID NO: 2):

Rabbit type X collagen: 5'-CCCAACACCAAGACACAGTT-3' (SEQ ID NO: 3) and

5'-ATCACCTTTGATGCCTGGCT-3' (SEQ ID NO: 4)

GAPDH

5'-GTCAAGGCCGAGAATGGAA-3' (SEQ ID NO: 5)

5'-GCTTCACCACCTTCTTGATG-3' (SEQ ID NO: 6)

### (2) Result

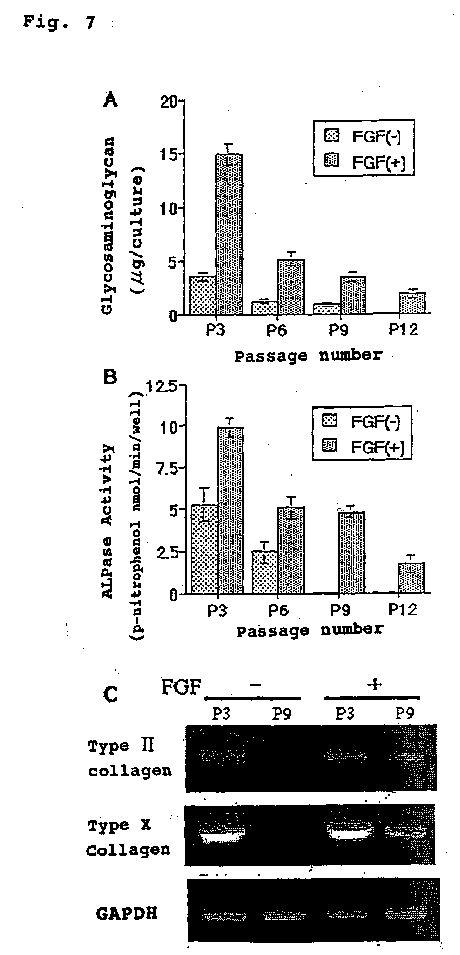

The amount of glycosaminoglycan, which is a cartilage matrix, of the mesenchymal stem cells in FGF(+) and FGF(-) cultures decreased with the increase in the passage number. However the amount of glycosaminoglycan of the mesenchymal stem cells in FGF(+) cultures was remarkably higher as compared with FGF(-) cultures (Fig. 7- A).

Similarly the alkaline phosphatase activity of FGF(+) and FGF(-) cultures decreased with the increase in the passage number, while the alkaline phosphatase activity of FGF(+) cultures was remarkably higher as compared with FGF(-) cultures (Fig. 7- B).

The amount of mRNA of type II collagen and type X collagen, which are marker molecules of chondrocyte, of FGF(+) and FGF(-) cultures decreased with the increase of passage number, while FGF(+) cultures contained higher amount of mRNA of type II collagen and type X collagen than FGF(-) cultures (Fig. 7- C).

These results indicate that FGF reinforces notably the chondrogenic differentiation potential of mesenchymal stem cells *in vitro* and represses the decrease in the differentiation potential along with passage.

### Example 6 Verification of osteogenic differentiation potential of cultured mesenchymal stem cells

The osteogenic differentiation potential of mesenchymal stem cells of FGF(+) or FGF(-) subcultures was verified in the following.

### (1) Method

Human mesenchymal stem cells were seeded at a high density (5000 cells/cm²) or a low density (1000 cells/cm²), subcultured in the presence of FGF or absence up to 3, 6 or 9 passages, and subsequently transferred to osteogenic differentiation medium described in Example 3 and cultured. Thus obtained cultures were analyzed for calcium content, alkaline phosphatase activity as well as the amount of mRNAs of bone sialoprotein (BSP), osteopontin and osteocalcin, which are genes characteristic of osteoblast.

The alkaline phosphatase activity in cell cultures was measured as in Example 5. The calcium content was measured according to the method of Gitelman (Anal. Biochem. 18 (1967), 521-531).

The amount of mRNAs of bone sialoprotein (BSP), osteopontin and osteocalcin was analyzed by RT-PCR method described in Example 5 using a set of the following primers.

### Primers

Human bone sialoprotein (BSP): 5'-CATTTTGGGAATGGCCTGTG-3' (SEQ ID NO: 7) and

5'-ATTGTCTCCTCCGCTGCTGC-3' (SEQ ID NO: 8)

Human osteopontin: 5'-CTAGGCATCACCTGTGCCATACC-3' (SEQ ID NO: 9) and

5'-CAGTGACCAGTTCATCAGATTCATC-3' (SEQ ID NO: 10)

Human osteocalcin: 5'-CCACCGAGACACCATGAGAG-3' (SEQ ID NO: 11) and

5'-CCATAGGGCTGGGAGGTCAG-3' (SEQ ID NO: 12)

GAPDH: 5'-GTCAAGGCCGAGAATGGGAA-3' (SEQ ID NO: 5) and

5'-GCTTCACCACCTTCTTGATG-3' (SEQ ID NO: 6)

### (2) Results

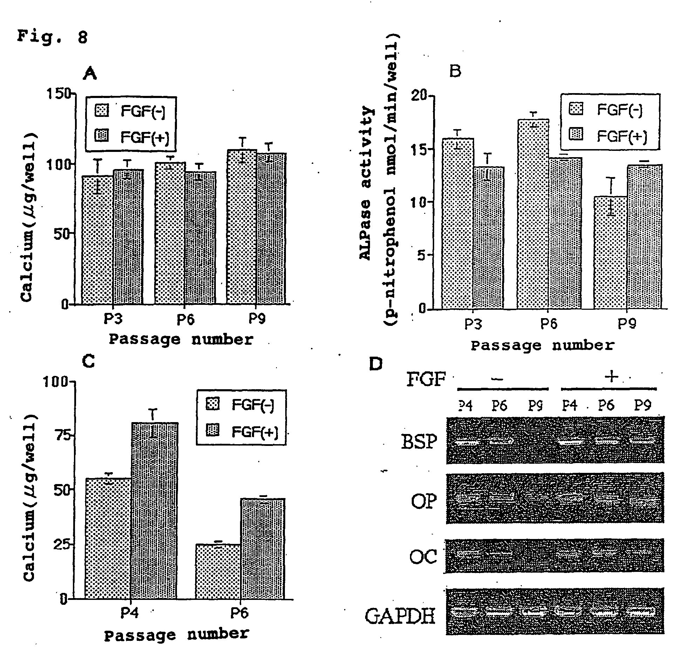

When the cells were seeded in a high density, the calcium content and alkaline phosphatase activity showed no significant difference regardless of the passage number, or of FGF(+) or FGF(-) (Fig. 8- A, C). However, when cells were seeded in a low density, the calcium content of FGF(+) was higher than FGF(-), and decreased along with passage number (Fig. 8- B).

The amount of mRNAs of bone sialoprotein (BSP), osteopontin and osteocalcin of FGF(+) and FGF(-) cultures showed no significant difference between 3^{rd} generation and 6^{th} generation, while the mRNA levels of FGF(-) mesenchymal stem cell cultures were decreased at 9^{th} generation (Fig. 8- D). These results indicate that FGF represses the decrease in osteogenic differentiation potential of mesenchymal stem cells *in vitro.*

Comparative Example Verification of adipogenic differentiation potential of cultured mesenchymal stem cells The adipogenic differentiation potential of mesenchymal stem cells of FGF(+) or FGF(-) subcultures was verified in the following.

Cells subcultured under FGF(+) or FGF(-) up to 4, 6, or 9 passages were transferred to the following adipogenic differentiation medium and cultured for 25 days to differentiate the mesenchymal stem cells into adipocytes.

### Adipogenic differentiation medium

αMEM medium (high glucose)
10% FBS
1µM Dexamethasone

### 0.2mM 3-Isobutyl-methyl-xanthine

The differentiation into adipocytes was detected by Oil Red staining and RT-PCR analysis of mRNA of PPAR-γ2, a differentiation marker molecule of adipocyte. RT-PCR was carried out as described in Example 5 using the following set of primers.

### Primers

Human PPAR-γ 2: 5'-CATTCTGGCCCACCAACTT-3' (SEQ ID NO: 13) and

5'-CCTTGCATCCTTCACAAGCA-3' (SEQ ID NO: 14)

GAPDH: 5'-GTCAAGGCCGAGAATGGGAA-3' (SEQ ID NO: 5) and

5'-GCTTCACCACCTTCTTGATG-3' (SEQ ID NO: 6)

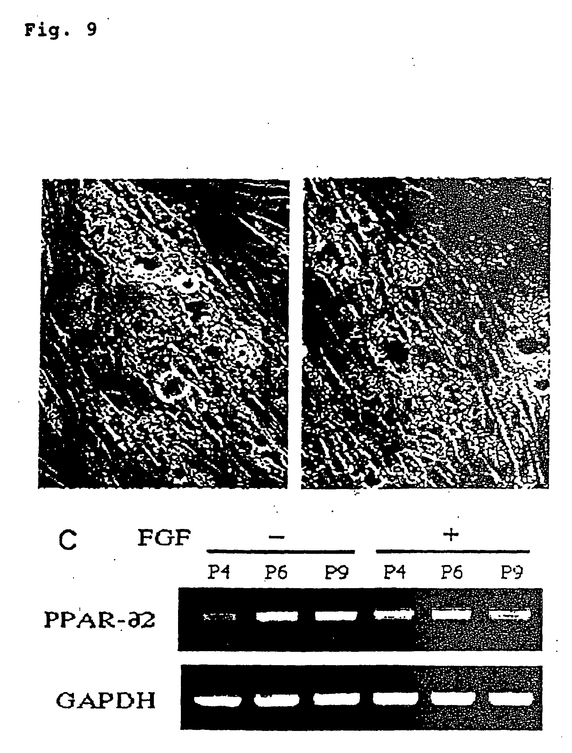

The mesenchymal stem cells of FGF(+) and FGF(-) subcultures were stained with Oil Red at the same level (Fig. 9- A, B). mRNA level of PPAR-γ2, a differentiation marker of adipocyte, was almost the same up to 9^{th} passage.

The results indicate that the existence or absence of FGF does not influence the differentiation of mesenchymal stem cells into adipocyte.

### Industrial applicability

The culture method of this invention enables to culture mesenchymal stem cells over 30 generations in contrast to the conventional culture method in which cell growth stopps around 15 generations. Available number of cell increases about 10⁵ to 10⁶-fold compared with the conventional culture methods. It is also possible to prevent the decrease in pluripotency along with the passage. Since the mesenchymal stem cells cultivated by this invention retain the differentiation potential to chondrocyte and osteoblast and have prolonged life span, they can provide large amounts of chondrocytes or osteocytes for transplantation of cartilage tissue or bone tissue. This is very important for the transplantation treatment of cartilage or bone.

### Brief description of drawings

Fig. 1 a graph which shows the cell growth in FGF-added group by [cell number/days in culture].
Fig. 2 is a graph which shows the cell growth in FGF-added group by [generation number/days in culture].
Fig. 3 is a microphotograph of the tissue before chondrogenic differentiation by the addition of bFGF.
Fig. 4 is a microphotograph of the tissue after chondrogenic differentiation by the addition of bFGF.
Fig. 5 ontains graphs showing the cell growth of the rabbit mesenchymal stem cells (derived from ilium (I), derived from tibia (T)) (A, B) in the presence of bFGF (1 ng/ml) or absence, and human mesenchymal stem cells (C).
Fig. 6 is to illustrate the chondrogenic differentiation potential of the mesenchymal stem cells by FGF, contains a series of microphotographs of cell pellets cultured in the presence of FGF (A-C) and absence (D), after 2 days (A), 4 days (B) and 8 days (C, D) from chondrogenic differentiation, and a series of microphotographs of cell pellets of 3, 6, 9 or 12 generations subcultured in the presence of FGF (E - H).
Fig. 7 is to illustrate the chondrogenic differentiation potential of the mesenchymal stem cells subcultured in FGF(+) or FGF(-), and shows the amount of glycosaminoglycan content (A), alkaline phosphatase activity (B) and expression of cartilage specific genes (type II collagen, type X collagen) (C).
Fig. 8 is to illustrate the osteogenic differentiation potential of the mesenchymal stem cells which subcultured in FGF(+) or FGF(-), and shows the calcium content of the mesenchymal stem cells seeded at a high density or low density (A, B), alkaline phosphatase activity (C), and expression of bone specific genes (bone sialoprotein (BSP), osteopontin (OP) and osteocalcin (OC)) (D).
Fig. 9 is to illustrate the adipogenic differentiation potential of the mesenchymal stem cells subcultured in FGF(+) or FGF(-), and shows microphotographs of the cells differentiated into adipocyte in FGF(+) (A) and FGF(-) (B), and expression of adipocyte specific gene (PPAR-γ2) (C).

## Claims

1. A method of culturing mammalian mesenchymal stem cells which is **characterized by** adding to a medium a substance which stimulates the proliferation potency of the mesenchymal stem cells.

2. The method of claim 1, wherein the above-mentioned substance is a fibroblast growth factor (FGF).

3. The method of claim 2, wherein FGF is added to a medium at a concentration of 0.04 to 10 ng/ml.

4. The method of claim 3, wherein FGF is added to a medium at a concentration of 0.1 to 1 ng/ml.

5. The method of any one of claims 1-4, wherein the mesenchymal stem cells are derived from human.

6. Mesenchymal stem cells retaining pluripotency obtained by a method of any one of claims 1-5.

7. The mesenchymal stem cells of claim 6 which have pluripotency to chondrocyte.

8. Use of FGF as a substance for stimulating the proliferation potency of mesenchymal stem cells.

9. Mesenchymal stem cells which retain the pluripotency in subculture over 15 generations.

10. The mesenchymal stem cells which retain the pluripotency in the subculture for 16 days or more.


SEQUENCE LISTING

<110> KATO Yukio

<120> Method for culturing bone marrow mesenchymal stem cells

<130> FP1021

<141> 2001-09-12

<150> JP2000-276971

<151> 2000-09-12

<160> 14

<210> 1

<211> 25

<212> DNA

<213> Artificial Sequence

<220>

<223> PCR primer

<400> 1

cataccggta agtggggcaa gactg 25

<210> 2

<211> 20

<212> DNA

<213> Artificial Sequence

<220>

<223> PCR primer

<400> 2

tgcccagttc aggtctctta 20

<210> 3
<211> 20
<212> DNA
<213> Artificial Sequence
<220>
<223> PCR primer
<400> 3
cccaacacca agacacagtt   20

<210> 4
<211> 20
<212> DNA
<213> Artificial Sequence
<220>
<223> PCR primer
<400> 4
atcacctttg atgcctggct   20

<210> 5
<211> 20
<212> DNA
<213> Artificial Sequence
<220>
<223> PCR primer
<400> 5
gtcaaggccg agaatgggaa   20

<210> 6

<211> 20

<212> DNA

<213> Artificial Sequence

<220>

<223> PCR primer

<400> 6

gcttcaccac cttcttgatg  20

<210> 7

<211> 20

<212> DNA

<213> Artificial Sequence

<220>

<223> PCR primer

<400> 7

cattttggga atggcctgtg  20

<210> 8

<211> 20

<212> DNA

<213> Artificial Sequence

<220>

<223> PCR primer

<400> 8

attgtctcct ccgctgctgc  20

<210> 9

<211> 23

<212> DNA
<213> Artificial Sequence
<220>
<223> PCR primer
<400> 9
ctaggcatca cctgtgccat acc 23

<210> 10
<211> 25
<212> DNA
<213> Artificial Sequence
<220>
<223> PCR primer
<400> 10
cagtgaccag ttcatcagat tcatc 25

<210> 11
<211> 20
<212> DNA
<213> Artificial Sequence
<220>
<223> PCR primer
<400> 11
ccaccgagac accatgagag 20

<210> 12
<211> 20
<212> DNA

<213> Artificial Sequence

<220>

<223> PCR primer

<400> 12

ccatagggct gggaggtcag   20

<210> 13

<211> 19

<212> DNA

<213> Artificial Sequence

<220>

<223> PCR primer

<400> 13

cattctggcc caccaactt   19

<210> 14

<211> 20

<212> DNA

<213> Artificial Sequence

<220>

<223> PCR primer

<400> 14

ccttgcatcc ttcacaagca   20